# EUROPÄISCHE PATENTSCHRIFT

(11) **EP 0 458 330 B1**
(45) Veröffentlichungstag und Bekanntmachung des Hinweises auf die Patenterteilung: **06.03.1996**
(21) Anmeldenummer: 91108347.5
(22) Anmeldetag: 23.05.1991
(51) Int. Cl.: G01N 21/82

(54) **Verfahren zur Bestimmung des von-Willebrand-Faktors**
Method for determining von-Willibrand factor
Procédé de détermination du facteur von-Willibrand

(30) Priorität: 25.05.1990 DE 4016885
(43) Veröffentlichungstag der Anmeldung: 27.11.1991
(73) Patentinhaber: BEHRINGWERKE Aktiengesellschaft, D-35001 Marburg (DE)
(72) Erfinder: Fickenscher, Karl, Dr., W-3550 Marburg (DE); Hissung, Alfred, Dr., W-3550 Marburg (DE)

(56) Entgegenhaltungen:
- EP-A- 0 078 451
- EP-A- 0 186 476
- FR-A- 2 634 886
- US-A- 4 640 896
- THROMBOS. DIATHES. HAEMORRH., Band 34, 1975, Stuttgart, DE, Seiten 306-308; MACFARLANE et al.: "A method for assaying von Willebrand factor (Ristocetin cofactor)"

## Beschreibung

Die Erfindung betrifft ein Verfahren zur funktionellen Bestimmung des von-Willebrand-Faktors durch Bestimmung der Sedimentationsgeschwindigkeit der Thrombozyten in Gegenwart von Ristocetin, das auch auf Zentrifugalanalysatoren angewendet werden kann.

Der von-Willebrand-Faktor (Faktor-VIII:vWF) ist der großmolekulare Anteil des Faktor VIII-Moleküls, und spielt durch seine Wechselwirkung mit Plättchen und Faktor VIIIC eine wichtige Rolle beim Ablauf der Gerinnung. Funktionsdefekte oder quantitative Mängel führen zu einer Blutungsneigung.

Das von-Willebrand-Syndrom ist eine erworbene oder erbliche Krankheit, die auf einen quantitativen oder qualitativen Mangel des F VIII:vWF zurückzuführen ist (Ruggeri, Z.M. & Zimmerman, T.S. 1987, Blood 70: 895-904). Die erbliche Form stellt wahrscheinlich die häufigste vererbte Gerinnungsstörung dar (Bowie, E.J.W., 1984, Clin. Lab. Med. 4, 303-317). Es sind mehrere Formen und Subformen der Erkrankung bekannt. Typ I ist dadurch gekennzeichnet, daß der F VIII:vWF in verminderter Menge vorliegt. Typ II ist durch qualitative Defekte gekennzeichnet, während die Menge normal ist. Man teilt Erkrankungen des Typs II noch in verschiedenen Subtypen auf, je nachdem ob Änderungen in der Multimerstruktur betroffen sind, oder ob es funktionelle Abnormalitäten sind.

Die Bestimmung des von-Willebrand-Faktors kann mit verschiedenen Testverfahren erfolgen, je nachdem welche Eigenschaft geprüft werden soll. Zur Bestimmung des Antigens stehen ELISA, Laurell-Immunelektrophorese und andere immunologischen Tests zur Verfügung (Goodall, A.H. et al. 1985, Brit. J. Haematol. 59: 565 -577; Sultan, Y. et al. 1984, Thromb. Haemost. 52: 250 -252).

Bekannt sind Verfahren zur funktionellen Bestimmung des Faktor VIII:vWF mittels stabilisierter oder mit in der Probe enthaltenen nativen Thrombocyten. Zu einer gerührten Suspension von nativen oder fixierten Plättchen wird in Anwesenheit von Ristocetin die Probe gegeben und die Rate der Änderung der Transmission aufgrund der ausgelösten Aggregation bestimmt (Macfarlane, D. E. et al., 1975, Thromb.Diathes.Haemorrh. 34: 306; EP-A-186476; Johnson et al., Annals NY Acad. Sci. 81: 227-235, 1981)

Thrombocyten haben sowohl im nativen Zustand, als auch in fixierter Form, wie sie für die Bestimmung des von-Willebrand-Faktors eingesetzt werden, die nachteilige Eigenschaft aufgrund ihres relativ hohen spezifischen Gewichts rasch zu sedimentieren. Daher wird in den bislang üblichen Testsystemen entweder in Küvetten unter Rühren auf Aggregometern oder in sehr geringen Schichtdicken auf Testplatten gearbeitet. Bisher konnte der von-Willebrand-Faktor nur manuell und damit auch nur mit den dadurch bedingten Ungenauigkeiten bestimmt werden.

Die FR-A-2 634 886 offenbart ein Verfahren zur Bestimmung des Ausmasses der Agglutination von Partikeln wie z.B. roten Blutkörperchen. Dabei wird die Probe durch Zentrifugation sedimentiert und die prozentuale Durchlässigkeit kontinuierlich verfolgt. Die Abweichung der Durchlässigkeitskurve von der durch Ursprung und Wendepunkt der Kurve bestimmten Geraden erlaubt Aussagen über das Ausmaß der Agglutination.

Der Erfindung lag die Aufgabe zugrunde, eine Methode zu finden, die eine vollautomatische, präzise Bestimmung des von-Willebrand-Faktors ermöglicht.

Gegenstand der Erfindung ist nun ein Verfahren zur Bestimmung des von-Willebrand-Faktors gemäß Anspruch 1.

Als Maß für die Sedimentationsgeschwindigkeit kann z. B. die Zeit bestimmt werden, die bis zur Veränderung der im Testansatz bestehenden Trübung auf einen vorgegebenen Wert vergeht.

Ebenfalls ermöglicht wird damit die Ermittlung der Funktionsfähigkeit von Blut-Plättchen, wenn plättchenreiches Plasma eingesetzt wird.

In einer bevorzugten Ausführung der Erfindung wird die Probe (200 µl plättchenarmes Plasma) in einer Kammer, ein Reagenz (aus 50 - 200 µl stabilisierten Plättchen und Ristocetin) in einer zweiten Kammer eines Zentrifugalanalysatorrotors vorgelegt. Probe und Reagenz werden durch Zentrifugation gemischt und dann die Reaktion bei 10 - 1000 x g bevorzugterweise bei 20 bis 200 x g optisch verfolgt. Die Messung kann bei jeder Wellenlänge, die die Feststellung einer Trübung erlaubt, durchgeführt werden. Beispielhaft soll hier eine Meßwellenlänge von 405 nm genannt werden; eine Wellenlänge, die auf entsprechenden Zentrifugalanalysatoren in der Regel bereits vorhanden ist. Zentrifugalanalysatoren sind dem Fachmann an sich bekannt.

Die Bestimmung kann bei 10-40 °C, bevorzugt bei 20-40 °C, ganz besonders bevorzugt bei 37 °C, erfolgen.

In einer bevorzugten Ausführung wird als Maß der Sedimentationsgeschwindigkeit die Zeit bestimmt, die vergeht von dem Start der Reaktion bis eine Änderung der optischen Dichte von z. B. 0.03 E erreicht ist. Die gefundene Zeit ist dem Gehalt an Faktor VIII:vWF proportional.

Ganz besonders bevorzugt ist die in dem Beispiel angegebene Ausführung.

Es wurde gefunden, daß mit dem beschriebenen Verfahren die Aktivität des in plättchenarmen Plasma vorliegenden von-Willebrand-Faktors sowohl sensitiv und präzise als auch einfach bestimmt werden kann. Der Test ist unempfindlich gegenüber F VIII:C (bis 400%). Es können kommerziell erhältliche Reagenzien (z. B. von Willebrand Reagenz (Behringwerke AG, D-3550 Marburg) eingesetzt werden.

Gegenüber den bekannten Verfahren zeichnet sich das erfindungsgemäße Verfahren durch seine Einfachheit, Sensitivität und leichte Automatisierbarkeit aus.

### Beispiel :

### Bestimmung des von-Willebrand-Faktors in einer PlasmaProbe

Zur Kalibrierung werden Verdünnungen eines plättchenarmen Normalplasma-Pools in phospatgepufferter, isotonischer Natriumchloridlösung von 100%, 75%, 50%, 25%, 10% und 0% hergestellt. Die Messung der Standards und der Proben erfolgt wie nachstehend beschrieben.

15 µl Probe (plättchenarmes Plasma) und 200 µl Reagenz (formalinfixierte Plättchen und Ristocetin) werden vorgelegt und durch Zentrifugation gemischt. Die Reaktion erfolgt bei 100 x g in einem Zentrifugalanalysator (ACL 300 der Firma IL). Die Messung wurde bei 405 nm durchgeführt.

Es würde sich aber jede andere Wellenlänge bei der die Trübung der Flüssigkeit durch die partikulären Blutplättchen feststellbar ist, ebenso eignen. Es wurde dann die Zeit bestimmt, um im Ansatz einen Abfall der optischen Dichte um 0.03 E zu erreichen. Die gefundene Zeit ist dem Gehalt an von-Willebrand-Faktor umgekehrt proportional.

## Patentansprüche

1. Verfahren zur quantitativen Bestimmung des von Willebrand Faktors, welches folgende Schritte einschließt:
a) Inkubation einer Plasmaprobe mit einem Reagenz, welches stabilisierte Plättchen und Ristocetin enthält;
b) Optische Bestimmung der Trübungsänderung, die eintritt, wenn die gebildeten Aggregate unter dem Einfluß einer definierten Zentrifugalbeschleunigung kontrolliert sedimentieren, und
c) Bestimmung der Menge des von Willebrand Faktors, in dem die Zeit bestimmt wird, die bis zur Veränderung der im Testansatz bestehenden Trübung auf einen vorgegebenen Wert vergeht.

2. Das Verfahren nach Anspruch 1, wobei die Zentrifugalbeschleunigung 10 x bis 1000 xg beträgt.

## Claims

1. A method for the quantitative determination of von Willebrand factor, which includes the following steps:
a) incubation of a plasma sample with a reagent which contains stabilized platelets and ristocetin;
b) optical determination of the change in the turbidity which occurs when the aggregates which are formed undergo controlled sedimentation under the influence of a defined centrifugal acceleration, and
c) determination of the amount of von Willebrand factor by determining the time which elapses until the turbidity existing in the assay mixture changes to a preset value.

2. The method as claimed in claim 1, wherein the centrifugal acceleration is from 10 × to 1000 × g.

## Revendications

1. Procédé pour la détermination quantitative du facteur von Willebrand, comprenant les étapes suivantes:
a) incubation d'un échantillon de plasma avec un réactif qui contient des plaquettes stabilisées et de la ristocétine;
b) détermination optique de la modification de trouble qui apparaît lorsque les agrégats formés se sédimentent de façon réglée, sous l'effet d'une accélération définie de centrifugation, et
c) détermination de la quantité au facteur von Willebrand, par détermination du temps qui se déroule jusqu'à la modification du trouble, présent dans le mélange d'essai, à une valeur préétablie.

2. Procédé selon la revendication 1, dans lequel l'accélération de centrifugation va de 10 à 1 000 g.
